(19) Europäisches Patentamt
European Patent Office
Office européen des brevets

(11) Veröffentlichungsnummer: **0 303 181 A2**

# (12) EUROPÄISCHE PATENTANMELDUNG

(21) Anmeldenummer: 88112679.1

(22) Anmeldetag: 04.08.88

(51) Int. Cl.⁴: C07D 295/12 , C07D 231/12 , C07D 261/08 , C07D 277/28 , C07D 249/04 , C07D 249/08 , C07D 271/10 , C07D 285/06 , C07D 285/10 , C07D 237/08 , C07D 239/26

(30) Priorität: 11.08.87 DE 3726632

(43) Veröffentlichungstag der Anmeldung:
15.02.89 Patentblatt 89/07

(84) Benannte Vertragsstaaten:
AT BE CH DE ES FR GB GR IT LI LU NL SE

(71) Anmelder: BOEHRINGER MANNHEIM GMBH
Sandhofer Strasse 116
D-6800 Mannheim 31(DE)

(72) Erfinder: Leinert, Herbert, Dr.
Essigkamm 11
D-6148 Heppenheim(DE)
Erfinder: Tsaklakidis, Christos, Dr.
Weberstrasse 24
D-6940 Weinheim(DE)
Erfinder: Sponer, Gisbert, Dr.
Lessingstrasse 13
D-6941 Laudenbach(DE)

## (54) 1,2-Diamino-Verbindungen, Verfahren zu ihrer Herstellung sowie Arzneimittel, die diese Verbindungen enthalten.

(57) Verbindungen der Formel I

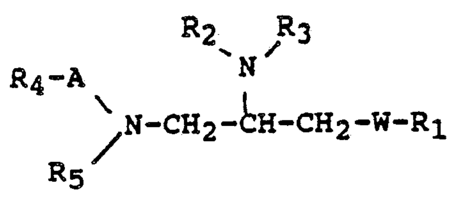

(I)

worin
$R_1$ Alkyl, Cycloalkyl oder Cycloalkylmethyl,
W Sauerstoff, Schwefel oder einen Valenzstrich
$R_2$ und $R_3$ Alkyl oder zusammen einen Ring
A einen Valenzstrich oder einen Alkylenrest,
$R_4$ einen gegebenenfalls substituierten mono- oder bicyclischen aromatischen Rest
und
$R_5$ einen substituierten Benzylrest, einen substituierten oder unsubstituierten Naphthylmethyl-Rest, einen unsubstituierten oder substituierten fünf- oder sechsgliedrigen oder bicyclischen Hetarylmethyl-Rest oder einen substituierten oder unsubstituierten Indan-1-yl- oder Indan-2-yl-Rest oder einen substituierten oder

EP 0 303 181 A2

unsubstituierten Tetralin-1-yl-oder Tetralin-2-yl-Rest bedeutetn,
wobei die Substituenten
Alkyl-, $C_2$-$C_6$-Alkenyl-, Alkoxy-, $C_2$-$C_6$-Alkenyloxy-, Hydroxyalkyl-, $C_2$-$C_6$-Alkylendioxy-, Hydroxy-alkoxy-, Alkoxy-alkoxy-, Alkylamino-, Dialkylamino-, Alkoxy-carbonyl-alkyloxy-, Alkylmercapto-, Alkylsulfinyl-, Alkylsulfonyl-, Alkylsulfonyloxy-, Carboxy-, Alkoxy-carbonyl-, Amino-carbonyl-, Mono- oder Dialkylamino-carbonyl-, Halogenalkyl-oder Cyano-Gruppen, sowie Halogenatome, wie Chlor, Brom oder Fluor sein können.
wobei
$R_5$ auch einen unsubstituierten Phenylrest bedeuten kann, wenn W einen Valenzstrich oder ein Schwefelatom bedeutet oder A eine Methylengruppe bedeutet, oder $R_4$ einen Naphthylrest, einen Tetralinylrest oder einen Indanylrest.
sowie deren pharmakologisch unbedenkliche Salze und Verfahren zur Herstellung von Verbindungen der Formel I, sowie Arzneimittel, die diese Verbindungen enthalten, zur Behandlung von Herz- und Kreislauferkrankungen.

## 1,2-Diamino-Verbindungen, Verfahren zu ihrer Herstellung sowie Arzneimittel, die diese Verbindungen enthalten

Die vorliegende Erfindung betrifft neue 1,2-Diamino-Verbindungen, deren pharmakologisch verträgliche Salze, ihre herstellung sowie diese enthaltende Arzneimittel.

Gegenstand der Erfindung sind neue 1,2-Diamino-Verbindungen der allgemeinen Formel I

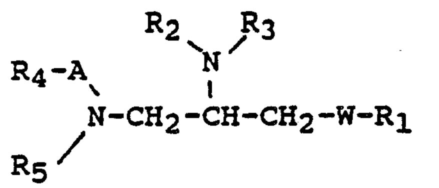

(I)

worin

$R_1$ einen geradkettigen oder verzweigten $C_1$-$C_{12}$-Alkylrest oder einen $C_3$-$C_7$-Cycloalkyl- oder $C_3$-$C_7$-Cycloalkylmethylrest

W ein Sauerstoffatom, ein Schwefelatom oder einen Valenzstrich bedeutet,

$R_2$ und $R_3$ gleich oder verschieden sein können und einen geradkettigen, verzweigten, gesättigten oder ungesättigten $C_1$-$C_6$-Alkylrest, der gegebenenfalls durch Hydroxy, $C_1$-$C_3$-Alkoxy oder $C_1$-$C_3$-Alkoxy-$C_1$-$C_3$-alkoxy substituiert sein kann,

bedeuten oder zusammen mit dem Stickstoffatom einen gesättigten oder ungesättigten Ring bilden, der noch weitere Heteroatome enthalten kann und zusätzlich gegebenenfalls durch eine niedrige Alkylgruppe, eine niedrige Alkoxygruppe oder ein Sauerstoffatom substituiert ist,

A einen Valenzstrich oder einen geradkettigen oder verzweigten Alkylrest mit 1-6, vorzugsweise 1-3 Kohlenstoffatomen bedeutet,

$R_4$ einen ein- oder mehrfach substituierten oder unsubstituierten mono- oder bicyclischen aromatischen Rest,

wobei die Substituenten

Alkyl-, $C_2$-$C_6$-Alkenyl-, Alkoxy-, $C_2$-$C_6$-Alkenyloxy-, Hydroxy-alkyl-, $C_2$-$C_6$-Alkylendioxy-, Hydroxy-alkoxy-, Alkoxy-alkoxy-, Alkylamino-, Dialkylamino-, Alkoxy-carbonyl-alkoxy-, Alkylmercapto-, Alkylsulfinyl-, Alkylsufonyl-, Alkylsulfonyloxy-, Carboxy-, Alkoxy-carbonyl-, Amino-carbonyl-, Mono- oder Dialkylamino-carbonyl-, Halogenalkyl-oder Cyano-gruppen sowie Halogenatome, wie Chlor, Brom oder Fluor sein können.

und

$R_5$ einen substituierten Benzylrest, einen substituierten oder unsubstituierten Naphthylmethyl-Rest, einen unsubstituierten oder substituierten fünf- oder sechsgliedrigen mono- oder bicyclischen Hetarylmethyl-Rest oder einen substituierten oder unsubstituierten Indan-1-yl- oder Indan-2-yl-Rest oder einen substituierten oder unsubstituierten Tetralin-1-yl-oder Tetralin-2-yl-Rest bedeutet,

wobei die Substituenten

Alkyl-, $C_2$-$C_6$-Alkenyl-, Alkoxy-, $C_2$-$C_6$-Alkenyloxy-, Hydroxyalkyl-, $C_2$-$C_6$-Alkylendioxy-, Hydroxy-alkoxy-, Alkoxy-alkoxy-, Alkylamino-, Dialkylamino-, Alkoxy-carbonyl-alkyloxy-, Alkylmercapto-, Alkylsulfinyl-, Alkylsulfonyl-, Alkylsulfonyloxy-, Carboxy-, Alkoxy-carbonyl-, Amino-carbonyl-, Mono- oder Dialkylamino-carbonyl-, Halogenalkyl-oder Cyano-Gruppen, sowie Halogenatome, wie Chlor, Brom oder Fluor sein können.

wobei

$R_5$ auch einen unsubstituierten Phenylrest bedeuten kann, wenn W einen Valenzstrich oder ein Schwefelatom bedeutet oder A eine Methylengruppe bedeutet, oder $R_4$ einen Naphthylrest, einen Tetralinylrest oder einen Indanylrest bedeutet

sowie deren pharmakologisch unbedenkliche Salze.

Der $C_1$-$C_{12}$-Alkylrest von $R_1$ bedeutet vorzugsweise Methyl, Ethyl, Propyl, Isopropyl, Butyl, Isobutyl, Isoamyl, Isohexyl, n-Hexyl, n-Octyl, n-Dodecyl, insbesondere die Isobutyl-, Isoamyl- und Isohexyl-Gruppe. Der $C_3$-$C_7$-Cycloalkylrest bedeutet in der Regel Cyclopentyl und Cyclohexyl.

$R_2$ und $R_3$ bedeuten vorzugsweise Methyl, Ethyl, Propyl, Allyl oder Methallyl. Ringe, die $R_2$ und $R_3$ zusammen mit dem Stickstoffatom, an das sie gebunden sind, bilden können, sind vorzugsweise der Pyrrolidin- und der Piperidinring, insbesondere der Pyrrolidinring. Die Heteroatome, die die Ringe enthalten

können, sind Stickstoff, Schwefel oder Sauerstoff.Es sind hierunter Ringe wie z. B. Piperazin, Morpholin und Thiomorpholin zu verstehen. Substituenten der vorstehend genannten Ringe sind insbesondere $C_1$-$C_3$-Alkyl- und $C_1$-$C_3$-Alkoxygruppen, wie z. B. Methyl, Ethyl oder Propyl oder Methoxy, Ethoxy oder Propoxy. Der Sauerstoff-Substituent stellt in der Regel mit dem C-Atom, an das er gebunden ist, eine Carbonylgruppe dar. Entsprechende Ringe sind z. B. der Pyrrolidinon- und Piperidinon-Ring.

$R_4$ bedeutet einen mono- oder bicyclischen aromatischen Rest. Hierunter sind Phenyl, Naphthyl, Tetralinyl- oder Indanyl zu verstehen. Die Alkylsubstituenten, allein oder in Verbindung mit anderen Resten, enthalten 1 - 6, vorzugsweise 1 - 4 C-Atome und bedeuten insbesondere Methyl.

Fünf- oder sechsgliedrige mono- oder bicyclische Hetarylmethyl-Reste enthalten vorzugsweise als Hetaryl-Komponenten Pyridyl-, Pyrimidinyl-, Pyrazinyl-, Thienyl-, Furanyl-, Pyrazolyl-, Imidazolyl-, Tetrazolyl-, Oxazolyl-, Isoxazolyl-, Thiazolyl-, Isothiazolyl-, Chinolinyl-, Isochinolinyl-, Benzofuranyl-, Benzothienyl-, Benzothiazolyl- oder Indolyl-Reste.

Die Alkylgruppen, allein oder in Verbindung mit anderen Resten, von Substituenten der Ringsysteme von $R_5$ enthalten 1 - 6, vorzugsweise 1- 4 C-Atome und bedeuten insbesondere Methyl. Die Substitution kann ein- oder mehrfach sein.

Die erfindungsgemäßen Verbindungen der allgemeinen Formel I können in an sich bekannter Weise dargestellt werden, indem man

a) eine Verbindung der allgemeinen Formel II,

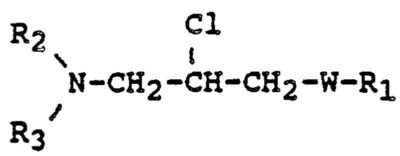

(II)

in der $R_1$, $R_2$, $R_3$ und W die oben genannten Bedeutungen besitzen, mit einer Verbindung der allgemeinen Formel III,

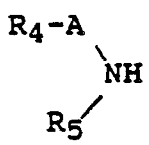

(III)

in der A, $R_4$ und $R_5$ die oben genannten Bedeutungen besitzen, umsetzt
oder

b) eine Verbindung der allgemeinen Formel IV,

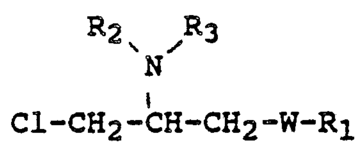

(IV)

in der $R_1$, $R_2$, $R_3$ und W die oben genannten Bedeutungen besitzen, mit einer Verbindung der allgemeinen Formel III umsetzt,
oder

c) eine Verbindung der allgemeinen Formel V,

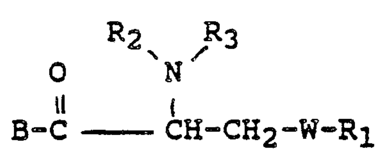

(V)

in der $R_1$, $R_2$, $R_3$ und W die oben angegebenen Bedeutungen besitzen und B ein Halogenatom oder eine Alkoxy-gruppe bedeutet, einer Amidbildungsreaktion mit einer Verbindung der allgemeinen Formel III unterzieht und die erhaltene Verbindung der allgemeinen Formel VI,

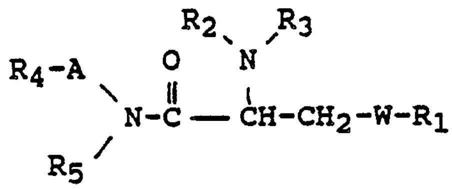 (VI)

in der $R_1$, $R_2$, $R_3$, $R_4$, $R_5$ und W die oben genannten Bedeutungen besitzen, einer Reduktion mit einem komplexen Hydrid oder Diboran unterwirft.

Die Umsetzung der Verbindungen der allgemeinen Formel II mit einer Verbindung der allgemeinen Formel III zu den erfindungsgemäßen Verbindungen der allgemeinen Formel I erfolgt, in an sich bekannter Weise, in einem inerten Lösungsmittel, wie Toluol, Xylol oder Dimethylformamid bei Raumtemperatur, bzw. zwischen 40° C und Rückflußtemperatur des Lösungsmittels in Gegenwart eines alkalischen Kondensationsmittels, z.B. Natriumhydrid oder Natriumamid.

Die Verbindungen der allgemeinen Formel II sind bekannt (vgl. EPS 0,138,684). Sie können in bekannter Weise dargestellt werden, daß man eine Verbindung der allgemeinen Formel VII

$R_1WH$ (VII)

in der $R_1$ die angegebenen Bedeutungen besitzt und
W ein Sauerstoffatom oder Schwefelatom bedeutet mit Epichlorhydrin zur Reaktion bringt und die erhaltene Verbindung der allgemeinen Formel VIII

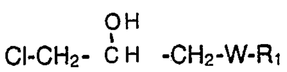 (VIII)

in Gegenwart von Natronlauge zu einer Verbindung der allgemeinen Formel IX

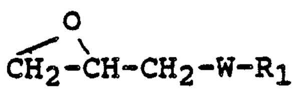 (IX)

umsetzt.

Umsetzungen der Verbindungen der allgemeinen Formel IX mit einem Amin der allgemeinen Formel X,

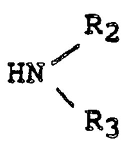 (X)

in der $R_2$ und $R_3$ die angegebenen Bedeutungen besitzen,
ergeben die Verbindungen der allgemeinen Formel XI

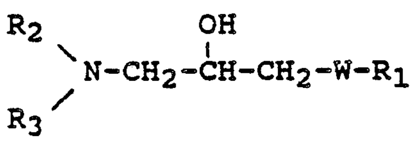 (XI)

Umsetzungen der Verbindungen der allgemeinen Formel XI mit Thionylchlorid in Gegenwart eines inerten Lösungsmittels ergeben die Verbindungen der allgemeinen Formel II.

Die Umsetzung der Verbindungen der allgemeinen Formel IV mit Verbindungen der allgemeinen Formel III zu den erfindungsgemäßen Verbindungen der allgemeinen Formel I erfolgt in einem inerten Lösungsmittel wie Toluol oder Xylol bei Temperaturen zwischen 40° C und Rückflußtemperatur des Lösungsmittels in Gegenwart eines alkalischen Kondensationsmittels wie Natriumhydrid oder Natriumamid.

Die Verbindungen der allgemeinen Formel IV können dargestellt werden, indem man eine Verbindung der allgemeinen Formel XII

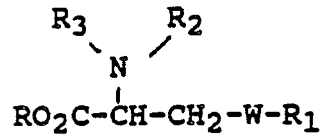

(XII)

in der $R_1$, $R_2$,$R_3$ und W die angegebenen Bedeutungen besitzen und R einen Alkylrest bedeutet, mit einem komplexen Hydrid, z. B. Lithiumaluminiumhydrid in einem inerten Lösungsmittel in an sich bekannter Weise zu einer Verbindung der allgemeinen Formel XIII

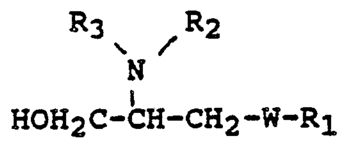

(XIII)

reduziert und diese in einem inerten Lösungsmittel mit Thionylchlorid zu einer Verbindung der allgemeinen Formel IV umsetzt.
Die Ausgangsverbindungen der allgemeinen Formel XII können nach dem in der DE-B 2802864 beschriebenen Verfahren hergestellt werden.

Die Umsetzung der Verbindungen der allgemeinen Formel V mit einer Verbindung der allgemeinen Formel III zu einer Verbindung der allgemeinen Formel VI sowie die Reduktion dieser Verbindungen zu den erfindungsgemäßen Verbindungen der allgemeinen Formel I erfolgt nach üblichen Verfahren, wie z. B. durch Reduktion mit $LiAlH_4$ oder Diboran in einem inerten Lösungsmittel, z. B. Ether oder THF.

Die Verbindungen der allgemeinen Formel V, in der B ein Halogenatom bedeutet, können hergestellt werden, indem man eine Verbindung der allgemeinen Formel XII einer Hydrolyse unterwirft und die erhaltene Verbindung der allgemeinen Formel XIV

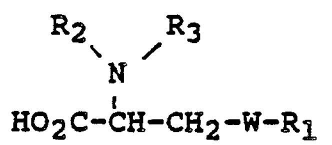

(XIV)

in der $R_1$, $R_2$, $R_3$ und W die oben angegebenen Bedeutungen besitzen mit einem Halogenierungsmittel, z. B. Thionylchlorid in einem inerten Lösungsmittel zur Reaktion bringt.

Die Verbindungen der allgemeinen Formel III können dargestellt werden durch Reduktion der entsprechenden Azomethin-Derivate oder durch Reduktion der entsprechenden Säureamide (vgl. Organikum, Organisch-Chemisches Grundpraktikum, 9. Auflage, Berlin 1970, S. 736).

Die erfindungsgemäßen Verbindungen der allgemeinen Formel I besitzen ein asymmetrisches Kohlenstoffatom.

Gegenstand der Erfindung sind daher auch Racemate und die optische aktiven Formen der erfindungsgemäßen Verbindungen der allgemeinen Formel I sowie Verfahren zu ihrer Herstellung. Die optisch aktiven Verbindungen können aus ihren racemischen Mischungen nach an sich bekannten Methoden über diasteromere Salze hergestellt werden. Zur Racematenspaltung können z. B. Weinsäure, Apfelsäure, Camphersäure, Camphersulfonsäure oder Dibenzoylweinsäure verwendet werden.

Zur Überführung der Verbindungen der allgemeinen Formel I in ihre pharmakologisch unbedenklichen Salze setzt man diese, vorzugsweise in einem organischen Lösungsmittel, mit der äquivalenten Menge einer anorganischen oder organischen Säure, z.B. Salzsäure, Bromwasserstoffsäure, Phosphorsäure, Cyclaminsäure, Schwefelsäure, Essigsäure, Salicylsäure, Citronensäure, Fumarsäure, Benzoesäure, Naphthoesäure, o-Acetoxybenzoesäure, Adipinsäure, Maleinsäure oder Oxalsäure um.

Die erfindungsgemäßen Verbindungen der allgemeinen Formel I besitzen wertvolle pharmakologische Eigenschaften. Sie zeichnen sich besonders durch eine gefäßrelaxierende Wirkung aus und können daher zur Therapie von Herz-/Kreislauf-Erkrankungen eingesetzt werden.

Die erfindungsgemäßen neuen Substanzen der allgemeinen Formel I und ihre Salze können in flüssiger oder fester Form enteral oder parenteral appliziert werden. Als Injektionsmedium kommt vorzugsweise

Wasser zu Anwendung, welches die bei Injektionslösungen üblichen Zusätze wie Stabilisierungsmittel, Lösungsvermittler oder Puffer enthält. Derartige Zusätze sind z.B. Tartrat- und Citratpuffer, Ethanol, Komplexbildner (wie Ethylendiamintetraessigsäure und deren nicht-toxischen Salze), hochmolekulare Polymere (wie flüssiges Polyethylenoxyd) zur Viskositätsregulierung. Feste Trägerstoffe sind z.B. Stärke, Lactose, Mannit, Methylcellulose, Talkum hochdisperse Kieselgelsäuren, höhermolekulare Fettsäuren (wie Stearinsäure), Gelatine, Agar-Agar, Calciumphosphat, Magnesiumstearat, tierische und pflanzliche Fette und feste hochmolekulare Polymere (wie z. B. Polyethylenglykole), für orale Applikation geeignete Zubereitungen können gewünschtenfalls Geschmacks- und Süßstoffe enthalten.

Neben den nachfolgenden aufgeführten Beispielen sind insbesondere die folgenden Verbindungen im Sinne der Anmeldung bevorzugt:

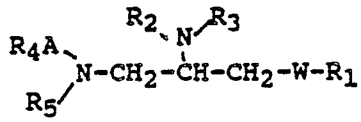

| $R_1$ | $R_2,R_3$ | $R_4$ | $R_5$ | W | A |
|---|---|---|---|---|---|
| $(CH_3)_2-CH-CH_2-$ | 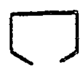 | 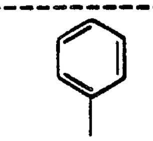 | 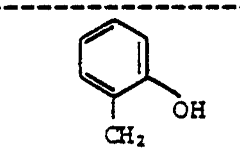 | O | - |
| $(CH_3)_2-CH-CH_2-$ | 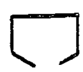 | 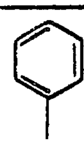 | 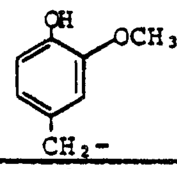 | O | - |
| $(CH_3)_2-CH-CH_2-$ | 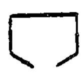 | 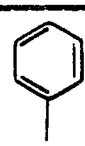 | 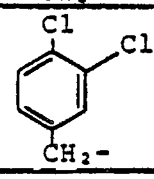 | O | - |
| $(CH_3)_2-CH-CH_2-$ | 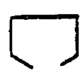 | 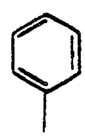 | 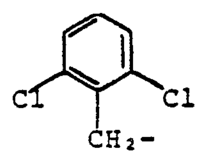 | O | - |
| $(CH_3)_2-CH-CH_2-$ | 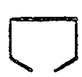 | 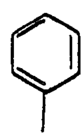 | 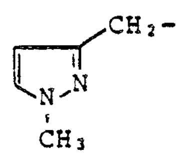 | O | - |
| $(CH_3)_2-CH-CH_2-$ | 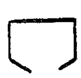 | 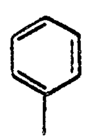 | 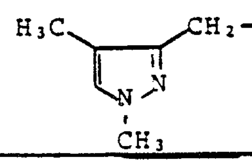 | O | - |
| $(CH_3)_2-CH-CH_2-$ | 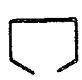 | 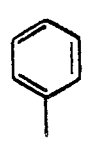 | 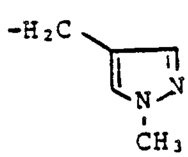 | O | - |
| $(CH_3)_2-CH-CH_2-$ | 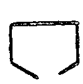 | 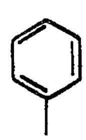 | 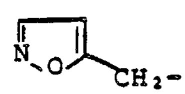 | O | - |

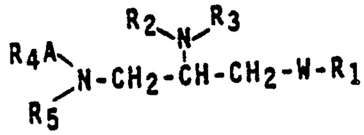

| $R_1$ | $R_2,R_3$ | $R_4$ | $R_5$ | W | A |
|---|---|---|---|---|---|
| $(CH_3)_2-CH-CH_2-$ | 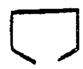 | 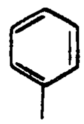 | 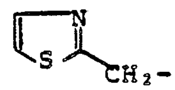 | O | - |
| $(CH_3)_2-CH-CH_2-$ | 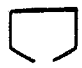 | 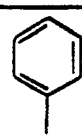 | 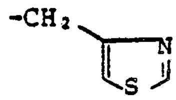 | O | - |
| $(CH_3)_2-CH-CH_2-$ | 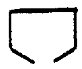 | 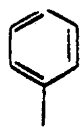 | 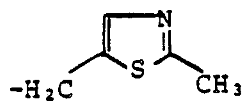 | O | - |
| $(CH_3)_2-CH-CH_2-$ | 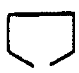 | 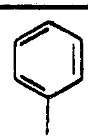 | 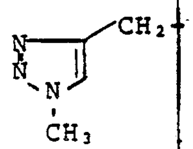 | O | - |
| $(CH_3)_2-CH-CH_2-$ | 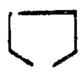 | 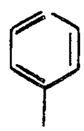 | 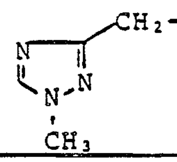 | O | - |
| $(CH_3)_2-CH-CH_2-$ | 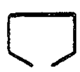 | 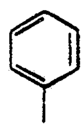 | 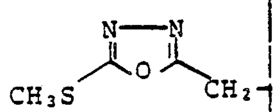 | O | - |
| $(CH_3)_2-CH-CH_2-$ | 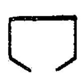 | 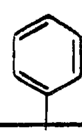 | 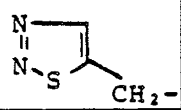 | O | - |
| $(CH_3)_2-CH-CH_2-$ | 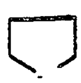 | 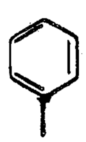 | 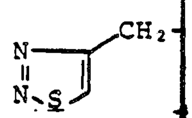 | O | - |

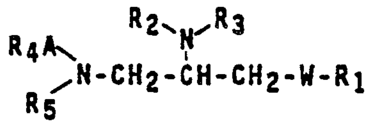

| $R_1$ | $R_2,R_3$ | $R_4$ | $R_5$ | W | A |
|---|---|---|---|---|---|
| $(CH_3)_2-CH-CH_2-$ | 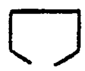 | 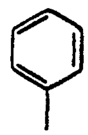 | 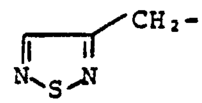 | O | - |
| $(CH_3)_2-CH-CH_2-$ | 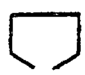 | 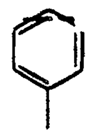 | 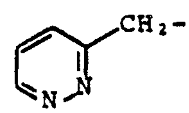 | O | - |
| $(CH_3)_2-CH-CH_2-$ | 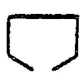 | 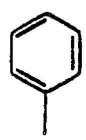 | 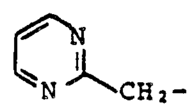 | O | - |
| $(CH_3)_2-CH-CH_2-$ | 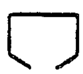 | 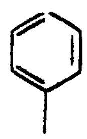 | 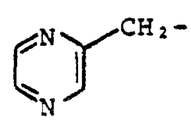 | O | - |
| $(CH_3)_2-CH-CH_2-$ | 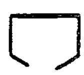 | 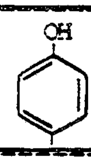 | 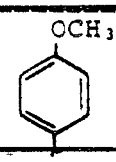 | O | - |
| $(CH_3)_2-CH-CH_2-$ | 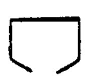 | 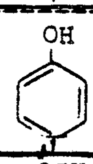 | 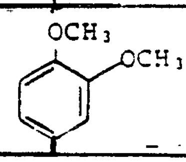 | O | - |
| $(CH_3)_2-CH-CH_2-$ | 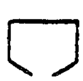 | 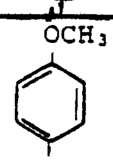 | 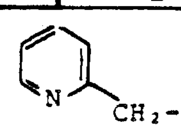 | O | - |

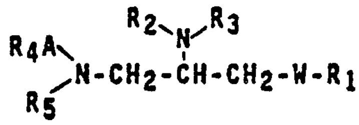

| $R_1$ | $R_2,R_3$ | $R_4$ | $R_5$ | W | A |
|---|---|---|---|---|---|
| $(CH_3)_2-CH-CH_2-$ | 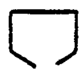 | 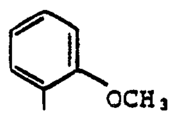 | 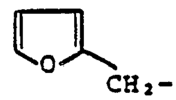 | O | - |
| $(CH_3)_2-CH-CH_2-$ | 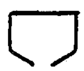 | 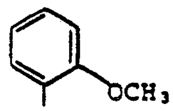 | 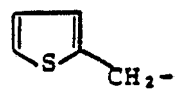 | O | - |
| $(CH_3)_2-CH-CH_2-$ | 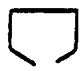 | 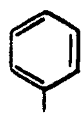 | 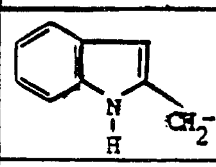 | O | - |
| $(CH_3)_2-CH-CH_2-$ | 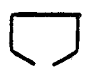 | 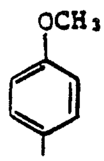 | 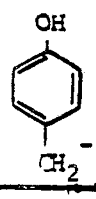 | O | - |
| $(CH_3)_2-CH-CH_2-$ | $-CH_2-CH_2-OCH_3$<br>$-CH_2-CH_2-OCH_3$ | 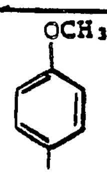 | 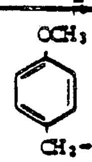 | O | - |
| $(CH_3)_2-CH-CH_2-$ | $-(CH_2)_2-O-(CH_2)_2-OCH_3$<br>$-(CH_2)_2-O-(CH_2)_2-OCH_3$ | 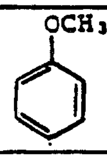 | 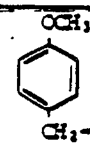 | O | - |
| $(CH_3)_2-CH-CH_2-$ | 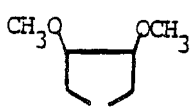 | 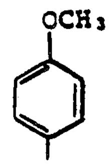 | 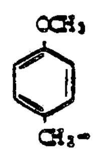 | O | - |

Beispiel 1:

2-(N-Pyrrolidino)-6-methyl-N-phenyl-N-benzyl-heptylamin-Oxalat

Zu einer Lösung von 5.5 g Natrium in 110 ml absol. Alkohol gibt man tropfenweise unter Rühren 34.8 g Diethylmalonat. Nach beendeter Zugabe erhitzt man noch eine Stunde am Rückfluß und gibt dann tropfenweise 41 g 4-Methyl-1-brompentan zu. Die Mischung wird noch 6 Stdn. am Rückfluß erhitzt und dann der Alkohol abdestilliert. Der Rückstand wird mit 100 ml konz. Kalilauge versetzt und die Mischung 2 Stdn. auf dem Wasserbad erwärmt. Dann wird abgekühlt und die Mischung mit 5 n Schwefelsäure sauer gestellt. Man erhitzt 3 Stdn. am Rückfluß, kühlt ab und extrahiert mit Essigester. Die Essigesterphase wird über Natriumsulfat getrocknet und eingedampft. Der Rückstand wird i. Vak. destilliert. Sdp. 120°C/2.5 mm. Die erhaltene Säure (40 g wird mit 50 ml Thionylchlorid versetzt und die Mischung 3 Stdn. am Rückfluß erhitzt. Dann wird eingedampft und der Rückstand unter Rühren bei 80°C tropfenweise mit 44 g Brom versetzt. Nach beendeter Zugabe erhitzt man 5 Stdn. weiter. Dann wird abgekühlt und das Reaktionsgemisch in 100 ml Alkohol eingetropft. Nach beendeter Zugabe erhitzt man 2 Stdn. am Rückfluß weiter.

Man kühlt und versetzt die Mischung tropfenweise unter Rühren mit 25 ml Pyrrolidin. Nach beendeter Zugabe erhitzt man noch 2 Stdn. am Rückfluß und dampft dann zur Trockene ein. Der Rückstand wird zur Reinigung an einer Kieselgelsäule chromatographiert. Man erhält 25 g eines öligen Rückstands von 6-Methyl-2-(N-pyrrolidino)-heptancarbonsäureethylester. Dieser wird in 100 ml absol. Tetrahydrafuran gelöst und die Mischung nach Zugabe von 8 g Lithiumaluminiumhydrid 3 Stdn. bei Raumtemperatur gerührt.

Das überschüssige Lithiumaluminiumhydrid wird durch Zugabe einer konz. Lösung von Aluminiumsulfat zersetzt und das Reaktionsprodukt mit Methylenchlorid extrahiert. Die Methylenchloridphase wird über Natriumsulfat getrocknet und eingedampft. Man erhält 15.4 g eines öligen Rückstandes von 6-Methyl-2-(N-pyrrolidino)-heptanol-1. 6 g dieser Verbindung werden in 20 ml Methylenchlorid gelöst und nach Zugabe von 8 g Thionylchlorid wird die Mischung 3 Stdn. am Rückfluß erhitzt. Dann wird eingedampft, der Rückstand in 20 ml Toluol gelöst, mit 7 g N-Benzylanilin und 2.8 g Natriumhydrid (50proz. ölige Suspension) versetzt und die Mischung 3 Stdn. am Rückfluß erhitzt. Dann wird abgekühlt, die Reaktionslösung mit Wasser versetzt und die organische Schicht abgetrennt. Diese wird über Natriumsulfat getrocknet und eingedampft. Der Rückstand wird zur Reinigung an einer Kieselgelsäule chromatographiert. (Elutionsmittel: Methylenchlorid/2 % Methanol). Die entsprechenden Säulenfraktionen werden eingedampft. Man erhält 7.2 g eines öligen Rückstandes. Dieser wird in Essigester gelöst und mit einer Lösung von Oxalsäure in Essigester versetzt. Die erhaltenen Kristalle werden abgesaugt und nochmals aus Essigester umkristallisiert. Man erhält 6.5 g der Titelverbindung vom Schmp. 125-126°C.

Beispiel 2:

2-(N-Pyrrolidino)-3-isobutoxy-N-phenyl-N-(2-methoxybenzyl)-propylamino-Oxalat

2.4 g 2-Methoxy-benzylanilin werden zusammen mit 2 g 2-Chlor-1-isobutoxy-3-(N-pyrrolidino)-propan in 20 ml absol. Toluol gelöst. Hierzu gibt man 0.9 g Natriumhydrid (50proz. ölige Suspension) und erhitzt die Mischung 3 Stdn. auf 100°C. Man kühlt ab, versetzt die Mischung mit Wasser und trennt die organische Schicht ab. Die wässrige Phase wird nochmals mit Toluol extrahiert. Die organischen Phasen werden vereinigt, über Natriumsulfat getrocknet und eingedampft. Der Rückstand wird durch Chromatographie an einer Kieselgelsäule gereinigt. (Fließmittel: Methylenchlorid/5 % Methanol). Die Säulenfraktionen werden eingedampft, der ölige Rückstand in Essigester gelöst und die Lösung mit einer Lösung von Oxalsäure in Essigester versetzt. Der Niederschlag wird abgesaugt und nochmals aus Essigester umkristallisiert. Man erhält 1.9 g der Titelverbindung.
Schmp. 108°C

Die folgenden Beispiele wurden analog dargestellt.

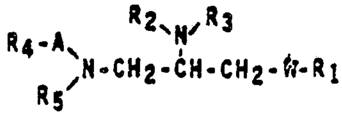

| Nr. | $R_1$ | $R_2,R_3$ | $R_4$ | $R_5$ | W | A | |
|---|---|---|---|---|---|---|---|
| 3 | $(CH_3)_2CH-CH_2-$ | 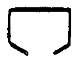 | 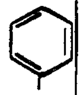 | 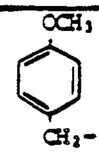 | 0 | - | Oxalat<br>130° C<br>Essigester |
| 4 | $(CH_3)_2CH-CH_2-$ | 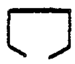 | 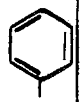 | 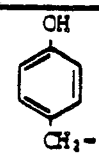 | 0 | - | Oxalat<br>106° C<br>Essigester |
| 5 | $(CH_3)_2CH-CH_2-$ | 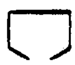 | 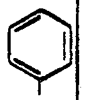 | 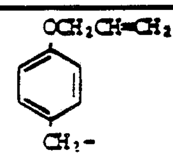 | 0 | - | Öl<br>m/e 422 |
| 6 | $(CH_3)_2CH-CH_2-$ | 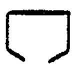 | 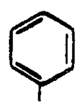 | 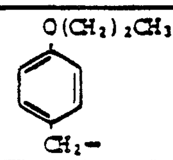 | 0 | - | Öl<br>m/e 424 |
| 7 | $(CH_3)_2CH-CH_2-$ | 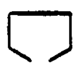 | 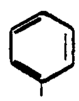 | 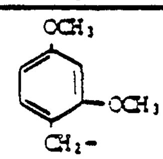 | 0 | - | Öl<br>m/e 426 |
| 8 | $(CH_3)_2CH-CH_2-$ | 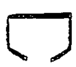 | 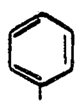 | 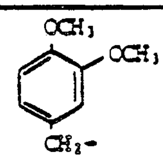 | 0 | - | Cyclamat<br>78 - 80° C<br>Ether |
| 9 | $(CH_3)_2CH-CH_2-$ | 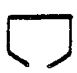 | 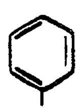 | 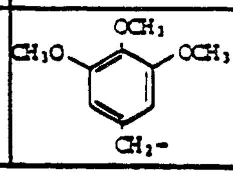 | 0 | - | Cyclamat<br>70° C<br>Ether |

| Nr. | $R_1$ | $R_2$,$R_3$ | $R_4$ | $R_5$ | W | A | |
|---|---|---|---|---|---|---|---|
| 10 | $(CH_3)_2CH-CH_2-$ | 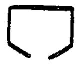 | 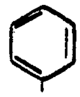 | 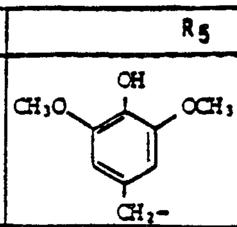 | 0 | - | Öl<br>m/e 442 |
| 11 | $(CH_3)_2CH-CH_2-$ | 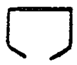 | 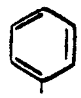 | 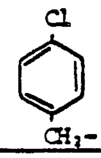 | 0 | - | Oxalat<br>140 - 141° C<br>Essigester |
| 12 | $(CH_3)_2CH-CH_2-$ | 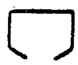 | 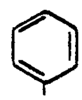 | 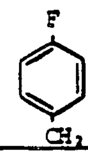 | 0 | - | Oxalat<br>144- 145° C<br>Essigester |
| 13 | $(CH_3)_2CH-CH_2-$ | 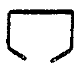 | 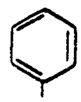 | 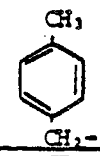 | 0 | - | Oxalat<br>135 - 136° C<br>Essigester |
| 14 | $(CH_3)_2CH-CH_2-$ | 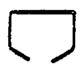 | 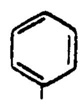 | 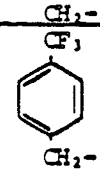 | 0 | - | Oxalat<br>129 - 130° C<br>Essigester |
| 15 | $(CH_3)_2CH-CH_2-$ | 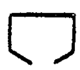 | 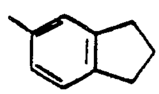 | 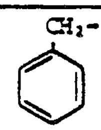 | 0 | - | Oxalat<br>137 - 138° C<br>Essigester |
| 16 | $(CH_3)_2CH-CH_2-$ | 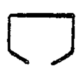 | 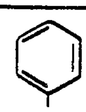 | 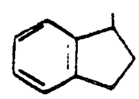 | 0 | - | Öl<br>m/e 392 |
| 17 | $(CH_3)_2CH-CH_2-$ | 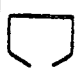 | 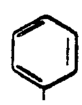 | 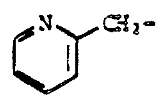 | 0 | - | Oxalat<br>144 - 145° C<br>Essigester |
| 18 | $(CH_3)_2CH-CH_2-$ | 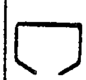 | 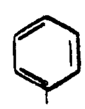 | 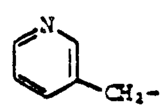 | 0 | - | Oxalat<br>143 - 144° C<br>Essigester |

| Nr. | $R_1$ | $R_2$,$R_3$ | $R_4$ | $R_5$ | W | A | |
|---|---|---|---|---|---|---|---|
| 19 | $(CH_3)_2CH-CH_2-$ | 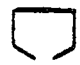 | 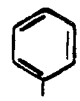 | 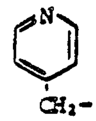 | 0 | - | Öl<br>m/e 367 |
| 20 | $(CH_3)_2CH-CH_2-$ | 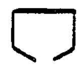 | 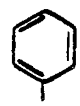 | 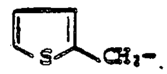 | 0 | - | Oxalat<br>142° C<br>Essigester |
| 21 | $(CH_3)_2CH-CH_2-$ | 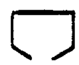 | 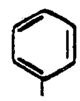 | 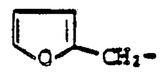 | 0 | - | Oxalat<br>132° C<br>Essigester |
| 22 | $(CH_3)_2CH-CH_2-$ | 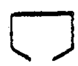 | 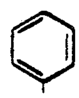 | 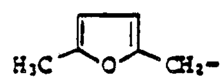 | 0 | - | Öl<br>m/e 460 |
| 23 | $(CH_3)_2CH-CH_2-$ | 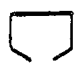 | 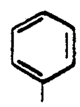 | 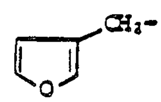 | 0 | - | Öl<br>m/e 446 |
| 24 | $(CH_3)_2CH-CH_2-$ | 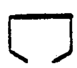 | 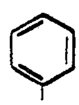 | 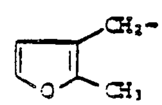 | 0 | - | Oxalat<br>128° C<br>Essigester |
| 25 | $(CH_3)_2CH-CH_2-$ | 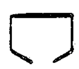 | 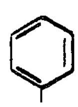 | 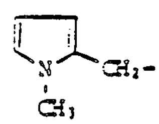 | 0 | - | Öl<br>m/e 369 |
| 26 | $(CH_3)_2CH-CH_2-$ | 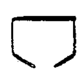 | 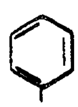 | 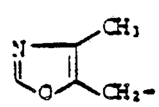 | 0 | - | Öl<br>m/e 371 |

| Nr. | $R_1$ | $R_2,R_3$ | $R_4$ | $R_5$ | W | A | |
|---|---|---|---|---|---|---|---|
| 27 | $(CH_3)_2CH-CH_2-$ | 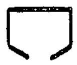 | 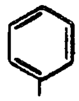 | 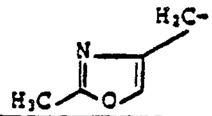 | 0 | - | Öl<br>m/e 371 |
| 28 | $(CH_3)_2CH-CH_2-$ | 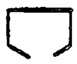 | 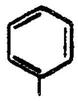 | 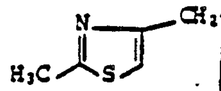 | 0 | - | Öl<br>m/e 387 |
| 29 | $(CH_3)_2CH-CH_2-$ | 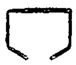 | 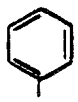 | 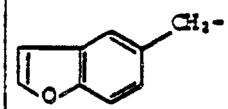 | 0 | - | Öl<br>m/e 406 |
| 30 | $(CH_3)_2CH-CH_2-$ | 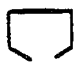 | 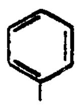 | 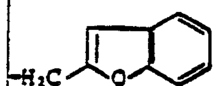 | 0 | - | Oxalat<br>143 - 144° C<br>Essigester |
| 31 | $(CH_3)_2CH-CH_2-$ | 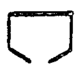 | 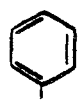 | 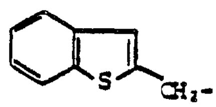 | 0 | - | Oxalat<br>124° C<br>Essigester |
| 32 | $(CH_3)_2CH-CH_2-$ | 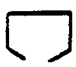 | 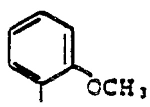 | 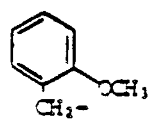 | 0 | - | Öl<br>m/e 426 |
| 33 | $(CH_3)_2CH-CH_2-$ | 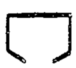 | 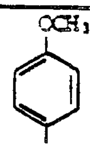 | 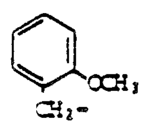 | 0 | - | Öl<br>m/e 426 |
| 34 | $(CH_3)_2CH-CH_2-$ | 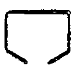 | 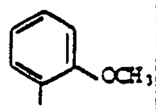 | 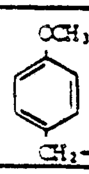 | 0 | - | Öl<br>m/e 426 |
| 35 | $(CH_3)_2CH-CH_2-$ | 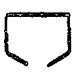 | 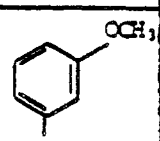 | 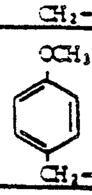 | 0 | - | Oxalat<br>95 - 96° C<br>Essigester |

| Nr. | $R_1$ | $R_2, R_3$ | $R_4$ | $R_5$ | W | A | |
|---|---|---|---|---|---|---|---|
| 36 | $(CH_3)_2CH-CH_2-$ | 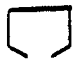 | 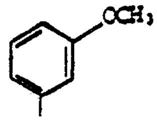 | 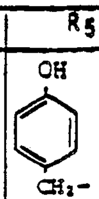 | 0 | - | Hydrochlorid 105 - 106° C Ether |
| 37 | $(CH_3)_2CH-CH_2-$ | 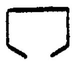 | 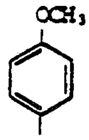 | 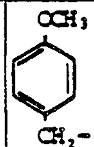 | 0 | - | Oxalat 94° C Essigester |
| 38 | $(CH_3)_2CH-CH_2-$ | 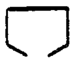 | 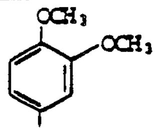 | 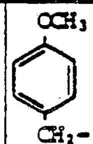 | 0 | - | Oxalat 104° C Essigester |
| 39 | $(CH_3)_2CH-CH_2-$ | 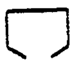 | 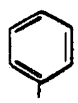 | 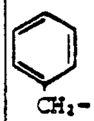 | 0 | $-CH_2-$ | Oxalat 90° C Essigester |
| 40 | $(CH_3)_2CH-CH_2-$ | 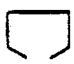 | 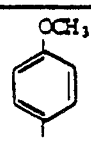 | 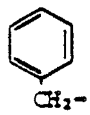 | 0 | $-CH_2-$ | Öl m/e 410 |
| 41 | $(CH_3)_2CH-CH_2-$ | 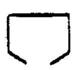 | 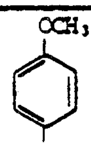 | 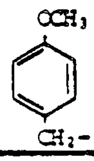 | 0 | $-CH_2-$ | Öl m/e 440 |
| 42 | $(CH_3)_2CH-CH_2-$ | 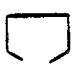 | 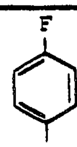 | 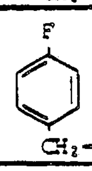 | 0 | - | Oxalat 97° C Essigester |
| 43 | $(CH_3)_2CH-CH_2-$ | 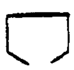 | 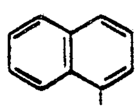 | 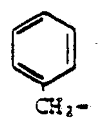 | 0 | - | Öl m/e 416 |
| 44 | $(CH_3)_2CH-CH_2-$ | 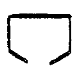 | 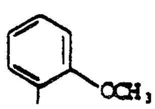 | 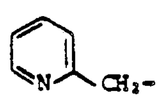 | 0 | - | Öl m/e 397 |

| Nr. | $R_1$ | $R_2,R_3$ | $R_4$ | $R_5$ | W | A | |
|---|---|---|---|---|---|---|---|
| 45 | $(CH_3)_2CH-CH_2-$ | 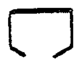 | 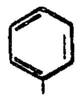 | 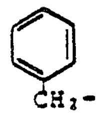 | S | - | Oxalat<br>97 - 99° C<br>Essigester |
| 46 | $(CH_3)_2CH-CH_2-$ | 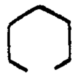 | 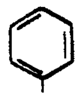 | 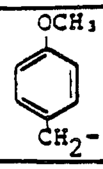 | O | - | Öl<br>m/e 410 |
| 47 | $(CH_3)_2CH-CH_2-$ | 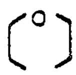 | 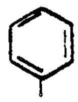 | 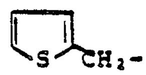 | O | - | Öl<br>m/e 388 |
| 48 | $(CH_3)_2CH-CH_2-$ | $-C_2H_5$<br>$-C_2H_5$ | 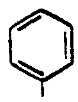 | 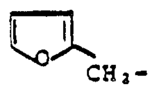 | O | - | Öl<br>m/e 358 |
| 49 | $(CH_3)_2CH-(CH_2)_2-$ | 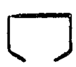 | 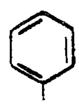 | 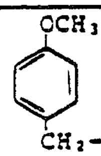 | O | - | Oxalat<br>136-137° C<br>Essigester |
| 50 | $(CH_3)_2CH-(CH_2)_3-$ | 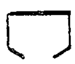 | 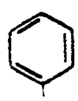 | 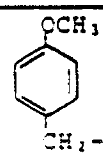 | O | - | Öl<br>m/e 424 |
| 51 | $(CH_3)_2CH-CH_2-$ | 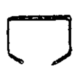 | 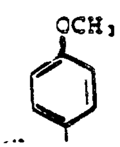 | 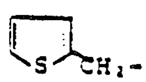 | O | - | Öl<br>m/e 402 |
| 52 | $(CH_3)_2CH-CH_2-$ | 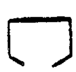 | 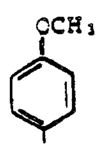 | 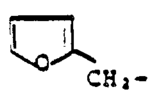 | O | - | Öl<br>m/e 386 |

| Nr. | $R_1$ | $R_2, R_3$ | $R_4$ | $R_5$ | W | A | |
|---|---|---|---|---|---|---|---|
| 53 | $(CH_3)_2CH-CH_2-$ | 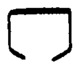 | 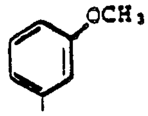 | 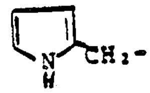 | 0 | - | Öl m/e 385 |
| 54 | $(CH_3)_2CH-CH_2-$ | 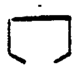 | 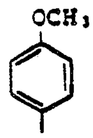 | 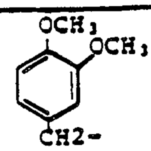 | 0 | - | Öl m/e 456 |
| 55 | $(CH_3)_2CH-CH_2-$ | 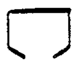 | 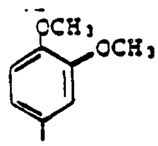 | 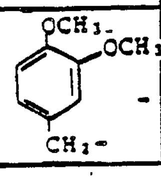 | 0 | - | Öl m/e 486 |
| 56 | $(CH_3)_2-CH-CH_2-$ | 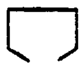 | 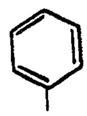 | 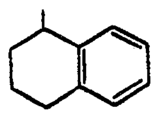 | 0 | - | Öl m/e 406 |
| 57 | $(CH_3)_2-CH-CH_2-$ | 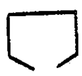 | 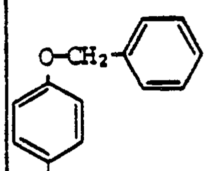 | 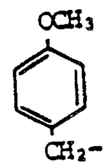 | 0 | - | Öl m/e 502 |
| 58 | $(CH_3)_2-CH-CH_2-$ | 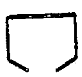 | 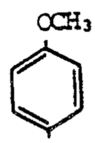 | 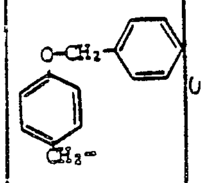 | 0 | - | Öl m/e 502 |
| 59 | $(CH_3)_2-CH-CH_2-$ | 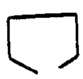 | 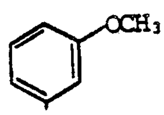 | 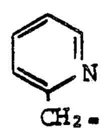 | 0 | - | Öl m/e 397 |
| 60 | $(CH_3)_2-CH-CH_2-$ | 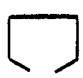 | 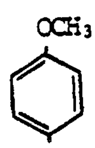 | 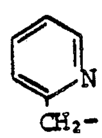 | 0 | - | Öl m/e 397 |

| Nr. | $R_1$ | $R_2, R_3$ | $R_4$ | $R_5$ | W | A | |
|---|---|---|---|---|---|---|---|
| 61 | $(CH_3)_2-CH-CH_2-$ | 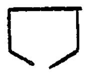 | 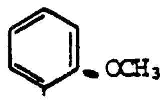 | 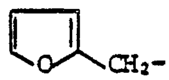 | 0 | - | Öl m/e 386 |
| 62 | $(CH_3)_2-CH-CH_2-$ | 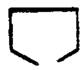 | 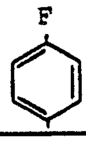 | 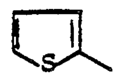 | 0 | - | Oxalat 140° C Essigester |
| 63 | $(CH_3)_2-CH-CH_2-$ | 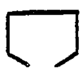 | 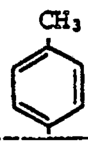 | 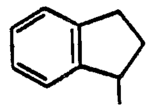 | 0 | - | Öl m/e 406 |
| 64 | $(CH_3)_2-CH-CH_2-$ | 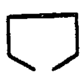 | 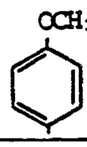 | 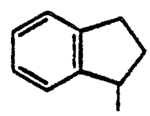 | 0 | - | Öl m/e 420 |
| 65 | $(CH_3)_2-CH-CH_3-$ | 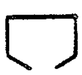 | 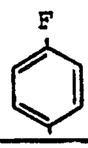 | 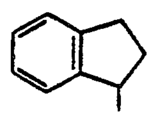 | 0 | - | Öl m/e 410 |
| 66 | $(CH_3)_2-CH-CH_2-$ | 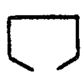 | 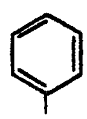 | 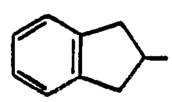 | 0 | - | Öl m/e 392 |
| 67 | $(CH_3)_2-CH-(CH_2)_2-$ | 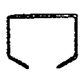 | 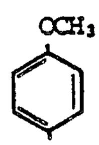 | 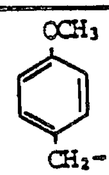 | 0 | - | Öl m/e 440 |
| 68 | $C_{10}H_{21}-$ | 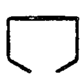 | 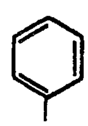 | 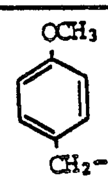 | 0 | - | Öl m/e 420 |
| 69 | $C_{10}H_{21}-$ | 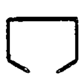 | 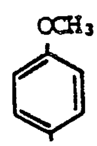 | 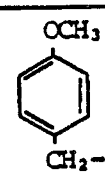 | 0 | - | Öl m/e 410 |

## Ansprüche

1. Verbindungen der Formel I

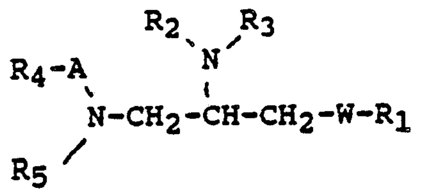

(I)

worin
$R_1$ einen geradkettigen oder verzweigten $C_1$-$C_{12}$-Alkylrest oder einen $C_3$-$C_7$ Cycloalkyl- oder $C_3$-$C_7$-Cycloalkylmethylrest
W ein Sauerstoffatom, ein Schwefelatom oder einen Valenzstrich bedeutet,
$R_2$ und $R_3$ gleich oder verschieden sein können und einen geradkettigen, verzweigten, gesättigten oder ungesättigten $C_1$-$C_6$-Alkylrest, der gegebenenfalls durch Hydroxy, $C_1$-$C_3$-Alkoxy oder $C_1$-$C_3$-Alkoxy-$C_1$-$C_3$-alkoxy substituiert sein kann,
bedeuten oder zusammen mit dem Stickstoffatom einen gesättigten oder ungesättigten Ring bilden, der noch weitere Heteroatome enthalten kann und zusätzlich gegebenenfalls durch eine niedrige Alkylgruppe, eine niedrige Alkoxygruppe oder ein Sauerstoffatom substituiert ist,
A einen Valenzstrich oder einen geradkettigen oder verzweigten Alkylrest mit 1-6, vorzugsweise 1-3 Kohlenstoffatomen bedeutet,
$R_4$ einen ein- oder mehrfach substituierten oder unsubstituierten mono- oder bicyclischen aromatischen Rest,
wobei die Substituenten
Alkyl-, $C_2$-$C_6$-Alkenyl-, Alkoxy-, $C_2$-$C_6$-Alkenyloxy-, Hydroxy-alkyl-, $C_2$-$C_6$-Alkylendioxy-, Hydroxy-alkoxy-, Alkoxy-alkoxy-, Alkylamino-, Dialkylamino-, Alkoxy-carbonyl-alkoxy-, Alkylmercapto-, Alkylsulfinyl-, Alkylsulfonyl-, Alkylsulfonyl-oxy-, Carboxy-, Alkoxy-carbonyl-, Amino-carbonyl-, Mono- oder Dialkylamino-carbonyl-, Halogenalkyl-oder Cyano-gruppen sowie Halogenatome, wie Chlor, Brom oder Fluor sein können.
und
$R_5$ einen substituierten Benzylrest, einen substituierten oder unsubstituierten Naphthylmethyl-Rest, einen unsubstituierten oder substituierten fünf- oder sechsgliedrigen mono- oder bicyclischen Hetarylmethyl-Rest oder einen substituierten oder unsubstituierten Indan-1-yl- oder Indan-2-yl-Rest oder einen substituierten oder unsubstituierten Tetralin-1-yl-oder Tetralin-2-yl-Rest bedeutet,
wobei die Substituenten
Alkyl-, $C_2$-$C_6$-Alkenyl-, Alkoxy-, $C_2$-$C_6$-Alkenyloxy-, Hydroxyalkyl-, $C_2$-$C_6$-Alkylendioxy-, Hydroxy-alkoxy-, Alkoxy-alkoxy-, Alkylamino-, Dialkylamino-, Alkoxy-carbonyl-alkyloxy-, Alkylmercapto-, Alkylsulfinyl-, Alkylsulfonyl-, Alkylsulfonyloxy-, Carboxy-, Alkoxy-carbonyl-, Amino-carbonyl-, Mono- oder Dialkylamino-carbonyl-, Halogenalkyl-oder Cyano-Gruppen, sowie Halogenatome, wie Chlor, Brom oder Fluor sein können.
wobei
$R_5$ auch einen unsubstituierten Phenylrest bedeuten kann, wenn W einen Valenzstrich oder ein Schwefelatom bedeutet oder A eine Methylengruppe bedeutet, oder $R_4$ einen Naphthylrest, einen Tetralinylrest oder einen Indanylrest.
sowie deren pharmakologisch unbedenklichen Salze und deren optische Isomeren.

2. Verfahren zur Herstellung von Verbindungen der Formel I
worin
$R_1$ einen geradkettigen oder verzweigten $C_1$-$C_{12}$-Alkylrest oder einen $C_3$-$C_7$ Cycloalkyl- oder $C_3$-$C_7$-Cycloalkylmethylrest
W ein Sauerstoffatom, ein Schwefelatom oder einen Valenzstrich bedeutet,
$R_2$ und $R_3$ gleich oder verschieden sein können und einen geradkettigen, verzweigten, gesättigten oder ungesättigten $C_1$-$C_6$-Alkylrest, der gegebenenfalls durch Hydroxy, $C_1$-$C_3$-Alkoxy oder $C_1$-$C_3$-Alkoxy-$C_1$-$C_3$-alkoxy substituiert sein kann,
bedeuten oder zusammen mit dem Stickstoffatom einen gesättigten oder ungesättigten Ring bilden, der noch weitere Heteroatome enthalten kann und zusätzlich gegebenenfalls durch eine niedrige Alkylgruppe, eine niedrige Alkoxygruppe oder ein Sauerstoffatom substituiert ist,

A einen Valenzstrich oder einen geradkettigen oder verzweigten Alkylrest mit 1-6, vorzugsweise 1-3 Kohlenstoffatomen bedeutet,
$R_4$ einen ein- oder mehrfach substituierten oder unsubstituierten mono- oder bicyclischen aromatischen Rest,
wobei die Substituenten
Alkyl-, $C_2$-$C_6$-Alkenyl-, Alkoxy-, $C_2$-$C_6$-Alkenyloxy-, Hydroxy-alkyl-, $C_2$-$C_6$-Alkylendioxy-, Hydroxy-alkoxy-, Alkoxy-alkoxy-, Alkylamino-, Dialkylamino-, Alkoxy-carbonyl-alkoxy-, Alkylmercapto-, Alkyl sulfinyl-, Alkylsulfonyl-, Alkylsulfonyloxy-, Carboxy-, Alkoxy-carbonyl-, Amino-carbonyl-, Mono- oder Dialkylamino-carbonyl-, Halogenalkyl-oder Cyano-gruppen sowie Halogenatome, wie Chlor, Brom oder Fluor sein können.
und
$R_5$ einen substituierten Benzylrest, einen substituierten oder unsubstituierten Naphthylmethyl-Rest, einen unsubstituierten oder substituierten fünf- oder sechsgliedrigen oder bicyclischen Hetarylmethyl-Rest oder einen substituierten oder unsubstituierten Indan-1-yl- oder Indan-2-yl-Rest oder einen substituierten oder unsubstituierten Tetralin-1-yl-oder Tetralin-2-yl-Rest bedeutet,
wobei die Substituenten
Alkyl-, $C_2$-$C_6$-Alkenyl-, Alkoxy-, $C_2$-$C_6$-Alkenyloxy-, Hydroxyalkyl-, $C_2$-$C_6$-Alkylendioxy-, Hydroxy-alkoxy-, Alkoxy-alkoxy-, Alkylamino-, Dialkylamino-, Alkoxy-carbonyl-alkyloxy-, Alkylmercapto-, Alkylsulfinyl-, Alkylsulfonyl-, Alkylsulfonyloxy-, Carboxy-, Alkoxy-carbonyl-, Amino-carbonyl-, Mono- oder Dialkylamino-carbonyl-, Halogenalkyl-oder Cyano-Gruppen, sowie Halogenatome, wie Chlor, Brom oder Fluor sein können.
wobei
$R_5$ auch einen unsubstituierten Phenylrest bedeuten kann, wenn W einen Valenzstrich oder ein Schwefelatom bedeutet oder A eine Methylengruppe bedeutet, oder $R_4$ einen Naphthylrest, einen Tetralinylrest oder einen Indanylrest.
sowie deren pharmakologisch unbedenklichen Salzen und deren optischen Isomeren,
dadurch gekennzeichnet, daß man in an sich bekannter Weise

a) eine Verbindung der allgemeinen Formel II,

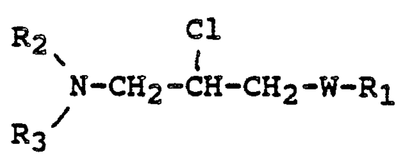

(II)

in der $R_1$, $R_2$, $R_3$ und W die oben genannten Bedeutungen besitzen, mit einer Verbindung der allgemeinen Formel III,

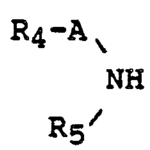

(III)

in der A, $R_4$ und $R_5$ die oben genannten Bedeutungen besitzen, umsetzt
oder

b) eine Verbindung der allgemeinen Formel IV,

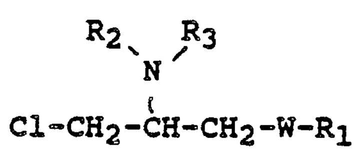

(IV)

in der $R_1$, $R_2$, $R_3$ und W die oben genannten Bedeutungen besitzen, mit einer Verbindung der allgemeinen Formel III umsetzt,
oder

c) eine Verbindung der allgemeinen Formel V,

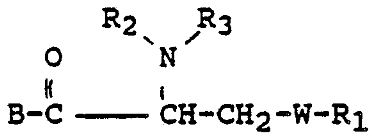

(V)

in der $R_1$, $R_2$, $R_3$ und W die oben angegebenen Bedeutungen besitzen und B ein Halogenatom oder eine Alkoxy-gruppe bedeutet, einer Amidbildungsreaktion mit einer Verbindung der allgemeinen Formel III unterzieht und die erhaltene Verbindung der allgemeinen Formel VI,

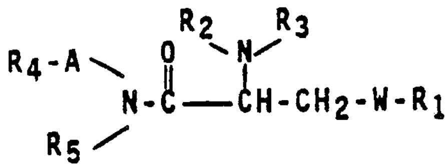

(VI)

in der $R_1$, $R_2$, $R_3$, $R_4$, $R_5$ und W die oben genannten Bedeutungen besitzen, einer Reduktion mit einem komplexen Hydrid oder Diboran unterwirft, und anschließend gewünschtenfalls die erhaltenen Verbindungen in ihre pharmakologisch unbedenkliche Salze überführt.

3. Arzneimittel, enthaltend eine Verbindung gemäß Anspruch 1 neben üblichen Träger- und Hilfsstoffen .

4. Verwendung von Verbindungen gemäß Anspruch 1, zur Behandlung von Herz- und Kreislauferkrankungen.